# EUROPEAN PATENT APPLICATION

(11) **EP 2 609 847 A1**
(43) Date of publication of application: **03.07.2013**
(21) Application number: 11845371.1
(22) Date of filing: 30.11.2011
(51) Int. Cl.: A61B 1/00

(54) **ENDOSCOPE**

(30) Priority: 01.12.2010 JP 2010268585; 21.02.2011 JP 2011034841
(71) Applicant: Olympus Medical Systems Corp., Tokyo 151-0072 (JP)
(72) Inventor: OMOTO, Keijiro, Tokyo 192-8512 (JP)
(74) Representative: Schicker, Silvia
(86) International application number: PCT/JP2011/077686
(87) International publication number: WO 2012/074013

(57) **Abstract**

An endoscope includes an insertion section, a first bending mechanism, a drive unit, a second bending mechanism, an operation-unit body, a grip, an angle mechanism, an operation member, and a control unit. The angle mechanism is provided on the operation-unit body and configured to set, to the first direction, a first operation input for bending the bending part in the first direction, and configured to transmit the first operation input to the first bending part. The operation member is provided on the grip or the operation-unit body and configured to set, to the second direction, an input direction of a second operation input for bending the bending part in the second direction.

## Description

### Technical Field

The present invention relates to an endoscope comprising a control device that can direct the bending part of, for example, the insertion section in the up/down direction (UD direction) and in the left/right direction (RL direction).

### Background Art

The endoscope has an insertion section to be inserted into a subject to enable the operator to observe and treat a target part in the subject, and a control device to be operated to bend the insertion section in the UD direction and RL direction. The control device has an UD angle knob for bending the insertion section in the UD direction, and an RKL angle knob for bending the insertion section in the RL direction. The operator may operate the UD angle knob and the RL angle knob to bend the insertion section, thereby to observe and treat the target part.

A skilled operator can manipulate both the UD angle knob and the RL angle knob with one hand, while holding the control device with the other hand.

However, an unskilled operator, no so much experienced in handing the endoscope, cannot operate both the UD angle knob and the RL angle knob with one hand, while holding the control device with the other hand, and therefore has to handle the UD angle knob with one hand, and the RL angle knob with the other hand.

In order to observe and treat a target part existing in a duct of a complex shape, such the large intestine that has many bent portions, the operator needs to turn the UD angle knob and the RL angle knob with, for example, the left hand only, while holding the sheath with the right hand to keep holding the insertion section in the lumen.

Any unskilled operator cannot help, but handle the UD angle knob and the RL angle knob with the hands, respectively. It is therefore very difficult for him or her to manipulate both the UD angle knob and the RL angle knob with, for example, the right hand, while holding the sheath with the right hand.

A technique of improving the operability of the insertion section in the UD direction and RL direction is disclosed in, for example, Jpn. UM Appln. KOKAI Publication No. 7-6882. Jpn. UM Appln. KOKAI Publication No. 7-6882 discloses an endoscope having an insertion section and a control section. The insertion section has a bending part that can be inserted into body cavities. The control section is configured to bend the insertion part. An extending base is provided, extending almost orthogonal to the control section. On the control section, a knob is provided, extending outwards and configured to bend the insertion section up and down (in the UD direction). On one side of the control section, a left-right (RL) direction bending switch is arranged. If turned by the operator, the knob bends the insertion in the up/down (UD) direction. If operated, the left-right (RL) direction bending switch drives a DC motor, bending the insertion part in the left/right direction (RL direction).

Jpn. Pat. Appln. KOKAI Publication No. 2004-8342 discloses an endoscope that has, at the upper end of the control section, an angle knob configured to bend the second bending part. To the angle know, a lever is secured, which can rotate together with the angle knob.

### Summary of Invention

The insertion section of the endoscope disclosed in Jpn. UT Appln. KOKAI Publication No. 7-6882 is manually bent in the up/down direction (UD direction) and automatically bent (using an electric motor) in the left/right direction (RL direction). To bend the insertion section in this manner, a knob and a left-right (RL) bending switch are provided on the control section. However, the knob and the left-right (RL) bending switch are not arranged to enable the operator to bend the insertion section in both the UD direction and the RL direction, while holding the control section with one hand.

Consequently, the operator of the endoscope disclosed in Jpn. UT Appln. KOKAI Publication No. 7-6882 bends the insertion section in the UD direction and the RL direction, while holding the control section with one hand, thereby to observe and treat the target part existing in, for example, a duct of a complex shape, such the large intestine having many bent portions. For the operator, however, it is hard to manipulate both the knob the left-right (RL) bending switch with, for example, the right hand only, while holding the sheath with the left hand.

With the endoscope disclosed in Jpn. Pat. Appln. KOKAI Publication No. 2004-8342, the operator can indeed operate the angle knob with one hand holding the control section if he or she manipulates, with the thumb, the lever that rotates as the angle knob is turned. However, the lever is used to bend the second bending part, not to bend the first bending part in the left-right (RL) direction. Inevitably, the operator cannot bend the first bending part in both the UD direction and the RL direction, while holding the control section with one hand. The UD angle knob is manipulated with the thumb in many cases. It is therefore very difficult to handle both the lever and the UD angle knob, in the case of the endoscope disclosed in Jpn. Pat. Appln. KOKAI Publication No. 2004-8342. Further, the lever of the endoscope disclosed in Jpn. Pat. Appln. KOKAI Publication No. 2004-8342 protrudes backward. Therefore, the operator may touch, by mistake, the lever with the thumb, to bend the second bending part unintentionally.

An object of the invention is to provide an endoscope enhanced in the operability in both the UD direction and the RL direction.

### Solution to Problem

An endoscope according to an aspect of the present invention comprises: an insertion section including a bending part capable of bending in a first direction and a second direction orthogonal to the first direction; a first bending mechanism configured to bend the bending part in the first direction; a drive unit configured to generate a drive force for bending the bending part in the second direction; a second bending mechanism configured to transmit the drive force to the bending part, thereby to bend the bending part in the second direction; an operation-unit body configured to bend the bending part; a grip formed integral with the operation-unit body and configured to be held with a hand; an angle mechanism provided on the operation-unit body and configured to set, to the first direction, a first operation input for bending the bending part in the first direction, and configured to transmit the first operation input to the first bending part if the first operation input is input; an operation member provided on the grip or the operation-unit body and configured to direct, in the second direction, an input direction of a second operation input for bending the bending part in the second direction; and a control unit configured to output a control signal for bending the bending part in the second direction, to the to the drive unit, in response to the second operation input.

The present invention provides an endoscope enhanced in the operability in both the UD direction and the RL direction.

### Brief Description of Drawings

FIG. 1 is a diagram showing the overall configuration of an endoscope apparatus according to a first embodiment of the invention;
FIG. 2 is a diagram showing the outer appearance of the control device of the endoscope of the apparatus;
FIG. 3 is a diagram showing the outer appearance of the dial used as RL operating member in the endoscope;
FIG. 4 is a diagram showing how the dial is inclined on the endoscope;
FIG. 5 is diagram showing the configuration of an endoscope according to a second embodiment of the invention;
FIG. 6 is diagram showing the configuration of an endoscope according to a third embodiment of the invention;
FIG. 7 is diagram showing the configuration of an endoscope according to a fourth embodiment of the invention;
FIG. 8 is a diagram showing the outer appearance of the endoscope, which an operator is holding with one hand;
FIG. 9 is a diagram showing the outer appearance of the dial used as RL operating member in the endoscope;
FIG. 10 is a diagram showing how the operator manipulates the dial;
FIG. 11 is a diagram showing the dial not being manipulated by the operator;
FIG. 12 is a diagram showing the outer configuration of an endoscope according to a fifth embodiment of the invention;
FIG. 13 is a plan view of the error preventing unit provided on the operation member of the endoscope according to the fifth embodiment;
FIG. 14 is a sectional view of the endoscope, taken along line Sa-Sa show in FIG. 13;
FIG. 15 is a diagram showing the overall configuration of an endoscope according to a sixth embodiment of the invention;
FIG. 16 is a plan view of the error preventing unit provided on the operation member of the endoscope according to the sixth embodiment;
FIG. 17 is a sectional view of the endoscope, taken along line Sb-Sb show in FIG. 16;
FIG. 18 is a diagram showing the dial provided on the endoscope of FIG. 15 and not manipulated by the operator;
FIG. 19 is a diagram showing the dial provided on the endoscope of FIG. 15 and being manipulated by the operator;
FIG. 20 is a diagram showing the outer appearance of an endoscope according to a seventh embodiment of the invention;
FIG. 21 is a diagram showing the outer appearance of an endoscope according to an eighth embodiment of the invention; and
FIG. 22 is a diagram showing the outer appearance of the endoscope of FIG. 21, which an operator is holding with one hand.

### Mode for Carrying Out the Invention

Embodiments of the invention will be described below, with reference to the accompanying drawing. It should be noted that the drawing is schematic, showing the components of each embodiment in a thickness-width relation different from the actual relation, and in a thickness ratio different from the actual ratio. It should also be noted that the thickness-width relation and the thickness ratio differ, between some views of the drawing.

### [First Embodiment]

The first embodiment of the invention will be described with reference to some views of drawing.

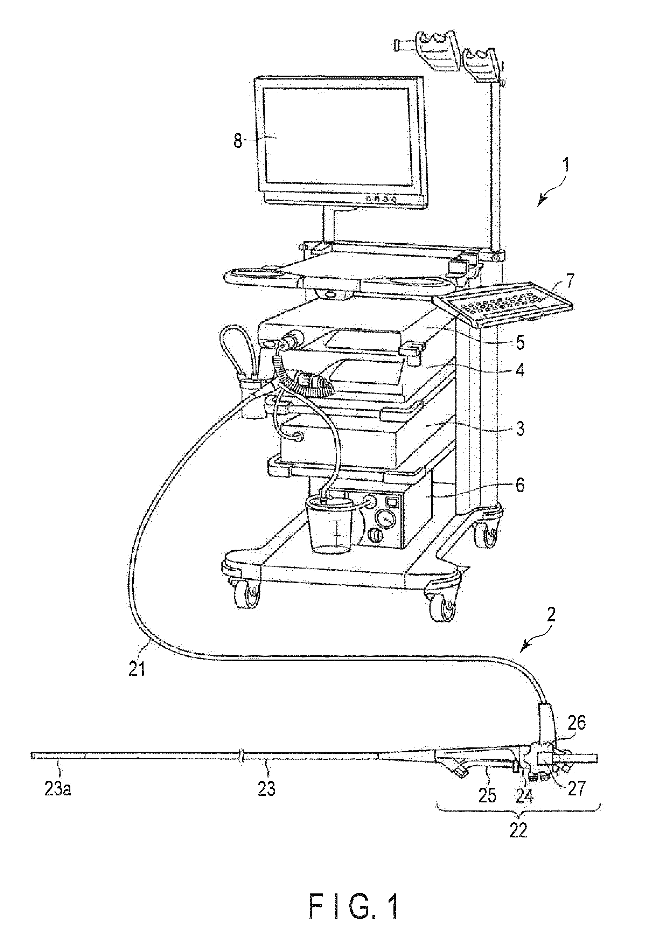

FIG. 1 is a diagram showing the overall configuration of the endoscope apparatus according to the first embodiment. The endoscope 1 has an endoscope 2, a control device 3, a light-source device 4, a photography device 5, a water-supplying device 6, a keyboard 7, and a monitor 8. The control device 3 controls the light-source device 4 to switches the device 4 on and off, causes the water-supplying device 6 to control the supply of water to the endoscope 2, processes the image of the subject the photography device 5 has photographed through the endoscope 2, and causes the monitor 8 to display the image of the subject.

The endoscope 2 comprises a universal cord 21, a operation device 22, and an insertion section 23. The universal cord 21 connects the endoscope 2 to the control device 3, light-source device 4, photography device 5 and a water-supplying device 6. If manipulated by an operator (user), such as a doctor, the operation device 22 directs the insertion section 23 up or down, or in the first direction (i.e., UD direction) and leftward or rightward, or in the second direction (i.e., RL direction).

The insertion section 23 is inserted into the subject. The insertion section 23 has a bending part 23a at the distal end inserted in the subject. The insertion section 23 includes the bending part 23a, which can be bent in the up/down direction (UD direction) and also in the left/right direction (RL direction) orthogonal to the UD direction. If controlled by the operation device 22, the insertion section 23 is bent, at the bending part 23a, in the UD direction and RL direction. As the operation device 22 is operated, the insertion section 23 is bent at the bending part 23a in the UD direction and RL direction. The insertion section 23 is made of flexible material.

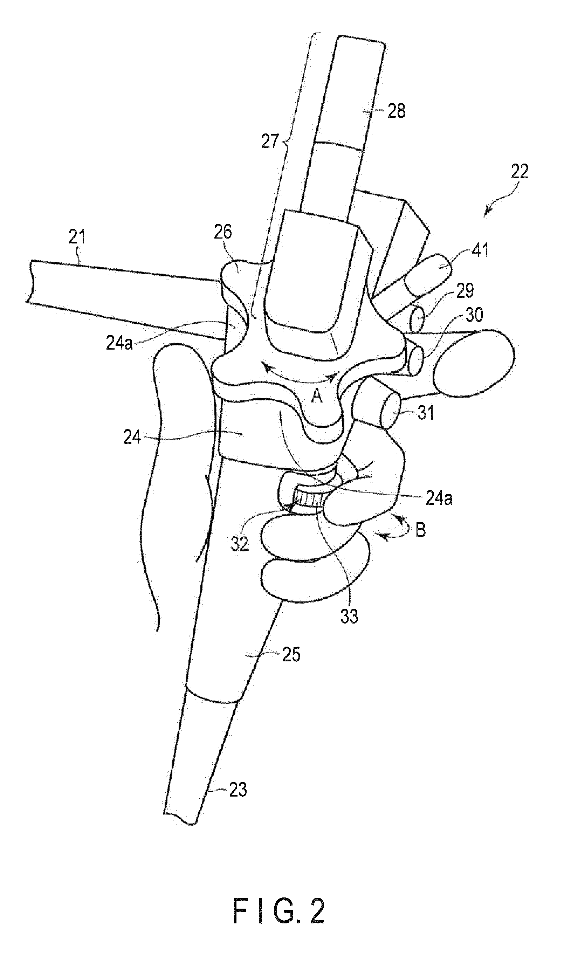

FIG. 2 shows the outer appearance of the operation device 22 of the endoscope 2. The operation device 22 comprises a operation-unit body 24 and a grip 25, which are formed integral and coaxial with each other. If manipulated, the operation-unit body 24 bends the bending part 23a. The grip 25 is formed integral with the operation-unit body 24. The operation-unit body 24 lies above the grip 25 while the operator is holding the operation device 22 with one hand. To the operation-unit body 24, the universal cord 21 is connected. To the grip 25, the insertion section 23 is coupled. The operation-unit body 24 and the grip 25 are so shaped that they are held in one palm while the operator is holding the grip 25 with one hand. For example, the operation device 22 is shaped, having its thickness gradually decreasing from the operation-unit body 24 toward the grip 25. The grip 25 is conical, having its diameter gradually reducing from the lower end of the operation-unit body 24 toward the insertion section 23.

The operation-unit body 24 has a first bending mechanism that is configured to bend the bending part 23a in the up-down direction (UD direction), or first direction. On the operation-unit body 24, a UD angle knob 26 and a UD release knob 41 are provided, which function as angle mechanism.

The UD angle knob 26 is mounted on the operation-unit body 24 so that the bending part 23a may be bent in the up/down direction (UD direction) in accordance with a first operation input (for bending the part 23a in the UD direction). If the operator generates a first operation input with any finger of one hand, while holding the grip 25 with the other hand, the UD angle knob 26 transmits the first operation input to the first bending mechanism. The first bending mechanism bends the bending part 23a in the up/down direction (UD direction).

The operator may turn the UD angle knob 26 in the direction of arrow A. Then, angle knob 26 bends the insertion section 23 in the UD direction. The UD angle knob 26 is provided on that side 24a of the operation-unit body 24, which is parallel to a plane defined by two lines along which the universal cord 21 and the grip 25 extend, respectively. The operator turns the UD angle knob 26 with, for example, the thumb and the forefinger or middle finger of one hand, while holding the grip 25 in the palm of the hand.

The UD release knob 41 fixes the UD angle knob 26 in position, preventing the knob 26 from rotating, and fixes or releases the angle by which to bend the bending part 23a of the insertion section 23.

On the axis of the UD angle knob 26, an RL drive unit 27 is provided. More specifically, the UD angle knob 26 and the RL drive unit 27 are mounted on the operation-unit body 24, the RL drive unit 27 overlapping the UD angle knob 26. An electric motor 28 for RL drive is coupled to the RL drive unit 27. If the electric motor 28 is driven, the insertion section 23 will be automatically bent in the RL direction.

On the operation-unit body 24, a switch 29, a suction button 30, and an air/water supply button 31 are provided. The switch 29, suction button 30 and air/water supply button 31 are arranged within such a small area that the operator can manipulate them all with, for example, the forefinger middle finger on one hand, while holding the grip 25 in the palm of the hand. The switch 29, suction button 30 and air/water supply button 31 are arranged ion a row, in the longitudinal direction of the operation-unit body 24.

An RL operation member 32 is provided on the upper part of the grip 25, for example a little below the boundary between the operation-unit body 24 and the grip 25. If turned by the operator in the direction of arrow B, the RL operation member 32 automatically bends the insertion section 23 in the RL direction. Hereinafter, the bending in the UD direction, achieved by turning the UD angle knob 26, will be referred to as main manipulation, and the bending in the RL direction, achieved by turning the RL operation member 32, will be referred to as sub-manipulation.

To be more specific, the RL operation member 32 is provided at that part of the grip 25, which lies a little below the UD angle knob 26. The RL operation member 32 is positioned in a manipulation area, so that the operator may turn it with any finger of, for example, the left hand, i.e., forefinger, middle finger, ring finger or small finger, while holding the grip 25 with the left hand. The manipulation area is an area in which the operator can turn the UD angle knob 26 with any finger of, for example, the left hand, (i.e., forefinger, middle finger, ring finger or small finger), while holding the grip 25 with the left hand.

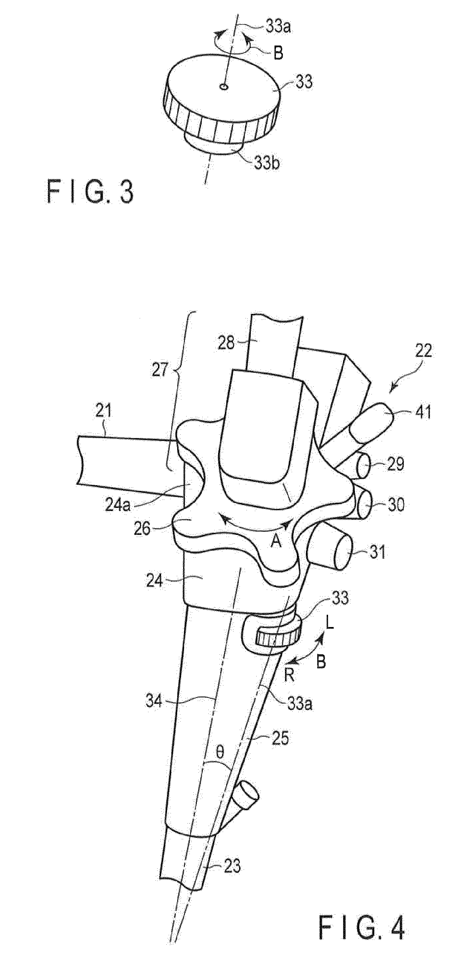

If turned by the operator, the RL operation member 32 drives the RL drive motor 28, which automatically bends the insertion section 23 in the UD direction. The RL drive motor 28 is a drive unit that generates a force to bend the bending part 23a to the left/right direction, i.e., second direction (RL direction). A second bending mechanism is provided to transmit the drive force of the RL drive motor 28 to the bending part 23a, thereby to bend the bending part 23a in the left/right direction (RL direction). That is, the RL operation member 32 has a dial 33. The dial 33 is shaped like, for example, a disc, as shown in FIG. 3. The dial 33 is provided, able to rotate in the direction of arrow B, around a shaft 33a passing through the centers of the circular surfaces of the dial 33, which oppose each other.

The RL operation member 32 is so arranged that a second operation input may be applied in the left/right direction (RL direction) to bend the bending part 23a if the operator manipulates the grip 25 or the operation-unit body 24 with any finger of one hand, while holding mainly the grip 25 with the hand and manipulating the angle mechanism including the UD angle knob 26.

In response to the second manipulation input, the control device 3 outputs a control signal to the RL drive motor 28, to bend the bending part 23a in the left/right direction (RL direction).

To the shaft 33a of the dial 33, a potentiometer 33b is coupled. The potentiometer 33b outputs an electric signal of a magnitude equivalent to the angle by which the dial 33 has been rotated in the direction of arrow B. The RL drive unit 27 receives the electric signal from the potentiometer 33b, and drives the RL drive motor 28 in accordance with the electrical magnitude of the electric signal. The insertion section 23 is thereby bent in the RL direction. The dial 33 may be rotated in the direction of arrow B, to adjust the angle by which to bend the insertion section 23 in the RL direction. The insertion section 23 is bent to the right (in R direction) if the dial 33 is rotated clockwise as viewed from above, or from the operation-unit body 24 toward the grip 25, and is bent to the left (in L direction) if the dial 33 is rotated counterclockwise.

As shown in FIG. 4, the shaft 33a of the dial 33 inclines by angle θ to the axis 34 common to the operation-unit body 24 and grip 25. Inclined by angle θ to the axis 34, the shaft 33a gradually leaves the axis 34 of the operation-unit body 24 and grip 25, as it extends from the grip 25 toward the operation-unit body 24. The inclination angle θ is set, ranging from 7° to 10°, for example. The optimal value for the inclination angle θ is, for example, 8.5°, particularly for the case where the operator rotates the dial 33 with, for example, the forefinger, middle finger, ring finger or small finger of one hand, while holding the grip 25 with the hand and manipulating the UD angle knob 26 with the thumb of the hand. The shaft 33a of the dial 33, which inclines at inclination angle θ may extend parallel to the sloping surface of the grip 25 shaped like a cone.

Since its shaft 33a is inclined by inclination angle θ, the dial 33 can rotate in the direction of arrow B extending along its circumference, or around the operation-unit body 24 and the axis 34 of the grip 25. While holding the grip 25 with one hand, e.g., left hand, the operator can easily rotate the dial 33 in the direction of arrow B with any finger of the left hand, i.e., forefinger, middle finger, ring finger or small finger. The dial 33 protrudes, in part, from the surface of the grip 25.

In response to the second operation input, the control device 3 outputs a control signal for bending the bending part 23a in the second direction.

The operator takes the endoscope 2 so configured, holding the grip 25 in the palm of his or her left hand. The operator may then rotate the UD angle knob 26 with, for example, the thumb in the direction of arrow A as shown in FIG. 2. As the UD angle knob 26 is so rotated, the insertion section 23 is bent in the UD direction.

The operator may further rotate the dial 33 of the RL operation member 32 with the middle finger in the direction of arrow B as shown in FIG. 2, while holding the grip 25 with one hand and manipulating the UD angle knob 26 with, for example, the thumb. In this case, the operator can rotate the dial 33 with, for example, the forefinger, ring finger or small finger, not with the middle finger. For example, the operator rotates the UD angle knob 26 in the direction of arrow A, with the thumb only. In this state, the operator can push either the suction button 30 or the air/water supply button 31 with, for example, the forefinger and can further rotate the dial 33 in the direction of arrow B with, for example, the middle finger.

If the dial 33 is rotated, the potentiometer 33b coupled to the shaft 33a of the dial 33 outputs an electric signal of the magnitude equivalent to the angle by which the dial 33 has been rotated in the direction of arrow B. The RL drive unit 27 receives the electric signal from the potentiometer 33b and drives the RL drive motor 28 in accordance with the electrical magnitude of the electric signal. The insertion section 23 is thereby bent in the RL direction.

Thus, even if the operator is not so much experienced in operating endoscopes, he or she can manipulate the UD angle knob 26 and also rotate the dial 33 of the RL operation member 32 in the same way as a skilled operator, while holding the operation device 22 with one hand. The operator, even if not so much skilled, can therefore the insertion section 23 not only in the UD direction, but also in the RL direction, at the same time. In this regard, it should be noted that the operator needs to manipulate the operation device 22 of the endoscope 2 with the left hand to bend the insertion section in both the UD direction and the RL direction, while holding the sheath with the right hand and thereby positioning the insertion section 23 in, for example, a duct of a complex shape, such the large intestine having many bent portions, thereby to observe and treat the target part existing in the duct.

In this endoscope apparatus, the insertion section 23 can be readily bent in both the UD direction and the RL direction. This enables an unskilled operator to bend the insertion section 23 in the UD direction and RL direction, in the same way as skilled operators do. The operator can therefore bend the insertion section 23 in both the UD direction and the RL direction with, for example, the hand only, while holding the sheath with the right hand and thereby positioning the insertion section 23 in a duct.

### [Second Embodiment]

The second embodiment of the invention will be described with reference to some other views of drawing. The components identical to those shown in FIG. 2 are designated by the same reference numbers and will not described in detail.

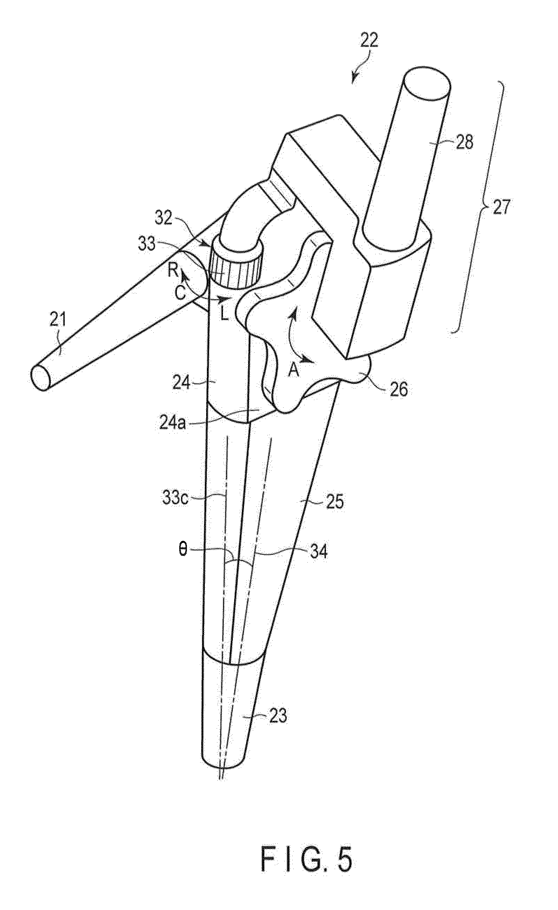

FIG. 5 shows the outer appearance of the operation device 22 of an endoscope. An RL operation member 32 is provided at the upper end of the operation-unit body 24 and on the left side of the UD angle knob 26. The RL operation member 32 is located in such an area that the operator can manipulate it, mainly with the thumb of, for example, the left hand, while holding the grip 25 in the palm of the left hand.

The RL operation member 32 may be provided at the upper end of the operation-unit body 24 and on the right side of the UD angle knob 26, not the left side thereof. In this case, the RL operation member 32 is located in such an area that the operator can manipulate it, mainly with the forefinger of, for example, the left hand, while holding the grip 25 in the palm of the left hand only. In said area, the operator can manipulate the UD angle knob 26, mainly with the forefinger of the left hand, while holding the grip 26 with the left hand only.

If so manipulated by the operator, the RL operation member 32 drives the RL drive motor 28, thereby automatically bending the insertion section 23 in the UD direction. The RL operation member 32 has a dial 33. The dial 33 is shaped like, for example, a disc, as shown in FIG. 3. The dial 33 is provided, able to rotate in the direction of arrow C, around a shaft 33c passing through the centers of the circular surfaces of the dial 33, which oppose each other.

To the shaft 33c of the dial 33, a potentiometer 33b is coupled. The potentiometer 33b outputs an electric signal of a magnitude equivalent to the angle by which the dial 33 has been rotated in the direction of arrow C. The RL drive unit 27 receives the electric signal from the potentiometer 33b, and drives the RL drive motor 28 in accordance with the electrical magnitude of the electric signal. The insertion section 23 is thereby bent in the RL direction. If the dial 33 is rotated clockwise as viewed from above, from the operation-unit body 24 toward the grip 25, the insertion section 23 will be bent the R direction. If the dial 33 is rotated counterclockwise, the insertion section 23 will be bent in the L direction.

The dial 33 has its shaft 33c inclined by angle θ to the axis 34 of the operation-unit body 24 and grip 25. Inclined by angle θ to the axis 34, the shaft 33c gradually leaves the axis 34 of the operation-unit body 24 and grip 25, as it extends from the grip 25 toward the operation-unit body 24. The inclination angle θ is set, ranging from 7° to 10°, for example. The optimal value for the inclination angle θ is, for example, 8.5°, particularly for the case where the operator rotates the dial 33, while holding the grip 25 with one hand.

The dial 33 protrudes, in part, from the surface of the operation-unit body 24.

The operator takes the endoscope 2 so configured, holding the grip 25 in the palm of his or her left hand. The operator may then rotate the UD angle knob 26 with, for example, the thumb in the direction of arrow A. As the UD angle knob 26 is so rotated, the insertion section 23 is bent in the UD direction.

The operator may further rotate the dial 33 of the RL operation member 32 with, for example, the thumb in the direction of arrow C as shown in FIG. 2, while holding the grip 25 with one hand. In this case, the operator can turn the UD angle knob 26 in the direction of arrow A, with the thumb only, can also rotate the dial 33 with the thumb in the direction of arrow C, and further can operate the switch 29, the suction button 30 or the air/water supply button 31 with, for example, the forefinger.

As the dial 33 is so rotated, the potentiometer 33b coupled to the shaft 33a of the dial 33 outputs an electric signal of the magnitude equivalent to the angle by which the dial 33 has been rotated in the direction of arrow B. The RL drive unit 27 receives the electric signal from the potentiometer 33b and drives the RL drive motor 28 in accordance with the electrical magnitude of the electric signal. The insertion section 23 is thereby bent in the RL direction.

Thus, even if the operator is not so much experienced in operating the endoscope 2, he or she can manipulate the UD angle knob 26 and also rotate the dial 33 of the RL operation member 32 in the same way as a skilled operator, while holding the operation device 22 with one hand, as with the endoscope 2 according to the first embodiment. The insertion section 23 can therefore be bent not only in the UD direction, but also in the RL direction.

In this endoscope apparatus, the insertion section 23 can be readily bent in both the UD direction and the RL direction. This enables an unskilled operator to bend the endoscope 2 in the UD direction and RL direction, in the same way as skilled operators do. The operator can therefore bend the insertion section 23 in both the UD direction and the RL direction with, for example, the left hand only, while holding the sheath with the right hand and thereby positioning the insertion section 23 in a duct.

### [Third Embodiment]

The third embodiment of the invention will be described with reference to some other views of drawing. The components identical to those shown in FIG. 2 are designated by the preference numbers and will not described in detail.

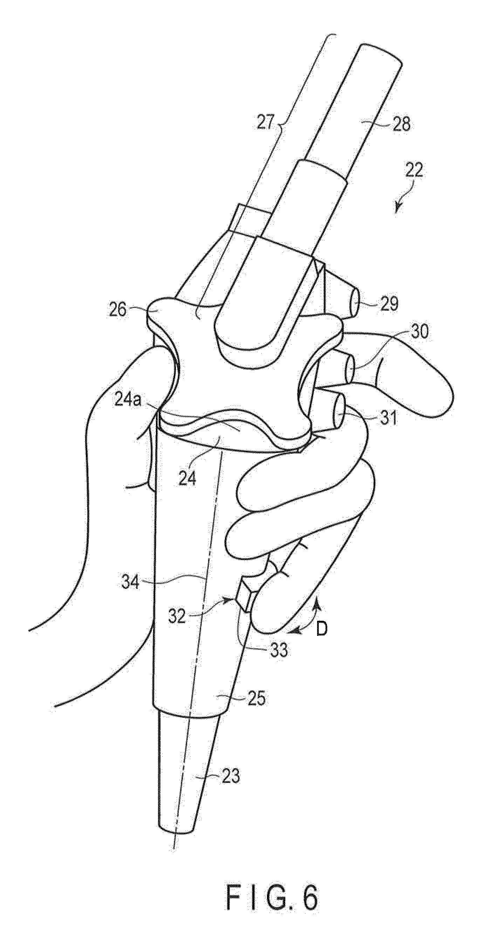

FIG. 6 shows the outer appearance of the operation device 22 of an endoscope 2. An RL operation member 32 is provided on the grip 25, at such a position as the operator can manipulate it with, for example, the small finger of the left hand, while holding the grip 25 with the left hand and turning the UD angle knob 26 with, for example, the thumb of the left hand. More specifically, the RL operation member 32 is provided on the grip 25, at the midpoint between the upper and lower ends of the grip 25, and below the switch 29, suction button 30 and air/water supply button 31 which are aligned in the vertical direction. Alternatively, the RL operation member 32 may be provided on that part of the grip 25, which lies between the row of the switch 29, suction button 30 and air/water supply button 31 and a frontal part of the operation-unit body 24.

If manipulated by the operator, the RL operation member 32 drives the RL drive motor 28, thereby automatically bending the insertion section 23 in the UD direction. The RL operation member 32 has a dial 33. To the shaft 33a of the dial 33, a potentiometer 33b is coupled. The potentiometer 33b outputs an electric signal of a magnitude equivalent to the angle by which the dial 33 has been rotated in the direction of arrow B.

The dial 33 protrudes, in part, from the surface of the operation-unit body 24.

The operator takes the endoscope 2 so configured, holding the grip 25 in the palm of his or her left hand. The operator may then rotate the UD angle knob 26 with, for example, the thumb in the direction of arrow A. As the UD angle knob 26 is so rotated, the insertion section 23 is bent in the UD direction.

While holding the grip 25 with one hand, the operator may rotate the dial 33 of the RL operation member 32 in the direction of arrow D with, for example, the small finger of the hand. Further, the operator can rotate the UD angle knob 26 with, for example, the thumb in the direction of arrow A, can turn the dial 33 of the RL operation member 32 in the direction of arrow D with, for example, the small finger, and can manipulate the switch 29, suction button 30 or air/water supply button 31 with, for example, the forefinger.

If the dial 33 is turned, the potentiometer 33b coupled to the haft 33a of the dial 33 outputs an electric signal of the magnitude equivalent to the angle by which the dial 33 has been rotated in the direction of arrow D. The RL drive unit 27 receives the electric signal from the potentiometer 33b and drives the RL drive motor 28 in accordance with the electrical magnitude of the electric signal. The insertion section 23 is thereby bent in the RL direction.

Thus, even if the operator is not so much experienced in operating the endoscope 2, he or she can manipulate the UD angle knob 26 and also rotate the dial 33 of the RL operation member 32 in the same way as a skilled operator, while holding the operation device 22 with one hand, as with the endoscope 2 according to the first embodiment. The insertion section 23 can therefore be bent not only in the UD direction, but also in the RL direction.

In this endoscope apparatus, the insertion section 23 can be readily bent in both the UD direction and the RL direction. This enables an unskilled operator to bend the endoscope 2 in the UD direction and RL direction, in the same way as skilled operators do. The operator can therefore bend the insertion section 23 in both the UD direction and the RL direction with, for example, the left hand only, while holding the sheath with the right hand and thereby positioning the insertion section 23 in a duct.

Each embodiment described above may be modified as will be described below. For example, the dial 33 used as RL operation member 32 may be provided on the operation-unit body 24. On the control body 24, the dial 33 may be provided at any position so long as the operator can turn the dial 33 with, for example, the forefinger, middle finger, ring finger or small finger of the left hand, while holding the grip 25 with the left hand.

### [Fourth Embodiment]

The fourth embodiment of the invention will be described with reference to some other views of drawing. The components identical to those shown in FIG. 2 are designated by the same reference numbers and will not described in detail.

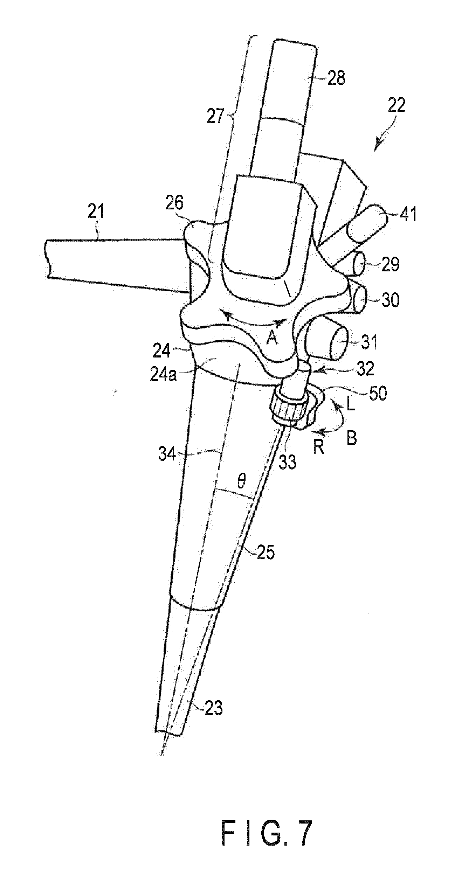

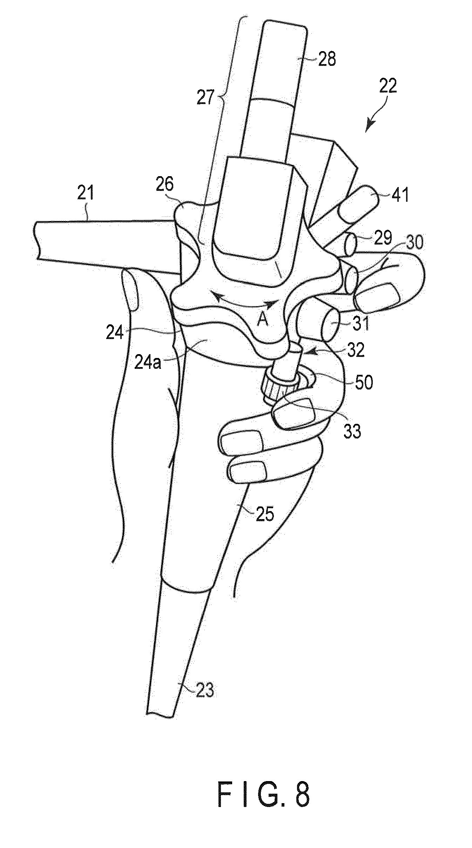

FIG. 7 shows the outer appearance of the operation device 22 of an endoscope 2. FIG. 8 shows the outer appearance of the operation device 22 an operator is holding with one hand.

The RL operation member 32 is positioned near the operating area of the UD angle knob 26, but never to interfere with the UD angle knob 26. The RL operation member 32 is provided on the control body 24, for example above the boundary between the control body 24 and the grip 25. If turned by the operator in the direction of arrow B, the RL operation member 32 automatically bends the bending part 23a of the insertion section 23 in the RL direction. The RL operation member 32 is arranged closer to the grip 25 than to the UD angle knob 26.

To be more specific, the RL operation member 32 is provided on that part of the control body 24, which lies below and near the UD angle knob 26. The RL operation member 32 is provided at the grip 25, at such a position that the operator can manipulate it with the forefinger, middle finger, ring finger or small finger of, for example, the left hand, while holding the grip 25 with the left hand and also turning the UD angle knob 26 with the thumb of the left hand.

If manipulated by the operator, the RL operation member 32 drives the RL drive motor 28. So driven, the RL drive motor 28 automatically bend the bending part 23a in the UD direction. The RL operation member 32 includes a dial 33 mounted on the grip 25.

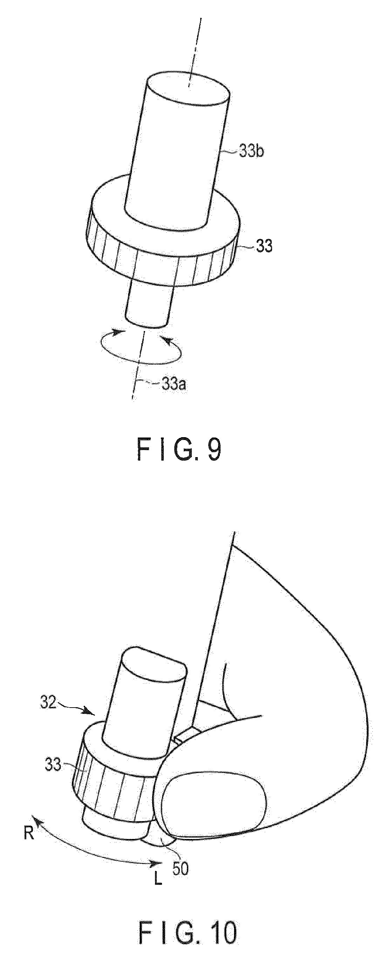

FIG. 9 shows the outer appearance of the dial 33 included in the RL operation member 32. The dial 33 is shaped like, for example, a disc. The dial 33 is provided, able to rotate in the direction of arrow B, around a shaft 33a passing through the centers of the circular surfaces of the dial 33, which oppose each other. A potentiometer 33b is coupled to the shaft 33a of the dial 33. The potentiometer 33b outputs an electric signal of a magnitude equivalent to the angle by which the dial 33 has been rotated in the direction of arrow B.

The RL drive unit 27 receives the electric signal from the potentiometer 33b and drives the RL drive motor 28 in accordance with the electrical magnitude of the electric signal. The insertion section 23 is thereby bent in the RL direction. The dial 33 may be rotated to adjust the angle by which to bend the bending part 23a in the RL direction. If the dial 33 is rotated clockwise as viewed from above, from the operation-unit body 24 toward the grip 25, the bending part 23a will be bent the R direction. If the dial 33 is rotated counterclockwise, the bending part 23a will be bent in the L direction.

As shown in FIG. 7, the dial 33 has its shaft 33a inclined by angle θ to the axis 34 of the operation-unit body 24 and grip 25, both shown in FIG. 9. Inclined by angle θ to the axis 34, the shaft 33c gradually leaves the axis 34 of the operation-unit body 24 and grip 25, as it extends from the grip 25 toward the operation-unit body 24.

The inclination angle θ is set, ranging from 7° to 10°, for example. The optimal value for the inclination angle θ is, for example, 8.5°, particularly for the case where the operator rotates the dial 33 with, for example, the forefinger, middle finger, ring finger or small finger of one hand, while holding the grip 25 with the left hand and turning the UD angle knob 26 with the thumb of the left hand.

The shaft 33a of the dial 33, which inclines at inclination angle θ, may extend parallel to the sloping surface of the grip 25 shaped like a cone. Since its shaft 33a inclines by inclination angle θ, the dial 33 can be rotated in the direction of arrow B, or in the circumferential direction, around the axis 34 of the operation-unit body 24 and grip 25. The operator can easily rotate the dial 33 in the direction of arrow B, with, for example, the forefinger, middle finger, ring finger or small finger of his or her left hand, while he or she is holding the grip 25 with the left hand. The dial 33 may protrude, in part, from the surface of the grip 25.

In the vicinity of the RL operation member 32, a projection 50 is arranged as an error preventing member. The projection 50 is provided on the operation-unit body 24, above, for example, the boundary between the operation-unit body 24 and the grip 25. The projection 50 is juxtaposed with the dial 33 of the RL operation member 32.

More precisely, the projection 50 intersects with that side of the operation-unit body 24, on which the UD angle knob 26 is arranged, and protrudes from the side on which the switch 29, suction button 30 and air/water supply button 31 are provided. The projection 50 lies below the air/water supply button 31 and away from the side 24a of the operation-unit body 24, never to prevent the dial 33 of the RL operation member 32 from being rotated.

The projection 50 has a recess in the middle part. The projection 50 is so shaped that the operator can touch it with, for example, the middle finger, ring finger or small finger of the left hand, while holding the grip 25 with the left hand.

The operator takes the endoscope 2 so configured, holding the grip 25 in the palm of his or her left hand. The operator may then rotate the UD angle knob 26 with, for example, the thumb in the direction of arrow A, as shown in FIG. 8. As the UD angle knob 26 is so rotated, the insertion section 23 is bent in the UD direction.

FIG. 10 shows how the operator rotates the dial 33. As shown in FIG. 10, the operator may rotate the dial 33 of the RL operation member 32 with, for example, the middle finger of one hand in the direction of arrow B, while holding the grip 25 with the left hand as shown in FIG. 8 and turning the UD angle knob 26 with, for example, the thumb. The operator can rotate the dial 33 with, for example, the forefinger, ring finger or small finger, instead of the middle finger. For example, the operator may manipulate the switch 29, suction button 30 or air/water supply button 31 with the forefinger and rotate the dial 33 in the direction of arrow B, while operating turning the UD angle knob 26 with the thumb in the direction of arrow A.

If the dial 33 is turned, the potentiometer 33b coupled to the haft 33a of the dial 33 outputs an electric signal of the magnitude equivalent to the angle by which the dial 33 has been rotated in the direction of arrow B. The RL drive unit 27 receives the electric signal from the potentiometer 33b and drives the RL drive motor 28 in accordance with the electrical magnitude of the electric signal. The insertion section 23 is thereby bent in the RL direction.

With this endoscope apparatus, the operator, even if not so much experienced in operating the endoscope 2, can manipulate the UD angle knob 26 and also rotate the dial 33 of the RL operation member 32 in the same way as a skilled operator, while holding the operation device 22 with one hand, as with the endoscope 2 according to the first embodiment. The insertion section 23 can therefore be bent not only in the UD direction, but also in the RL direction. In this regard, it should be noted that the operator needs to manipulate the operation device 22 of the endoscope 2 with the left hand to bend the insertion section in both the UD direction and the RL direction, while holding the sheath with the right hand and thereby positioning the insertion section 23 in, for example, a duct of a complex shape, such the large intestine having many bent portions, thereby to observe and treat the target part existing in the duct.

With this endoscope 2, the insertion section 23 can be readily bent in both the UD direction and the RL direction. This enables an unskilled operator to bend the endoscope 2 in the UD direction and RL direction, in the same way as skilled operators do. The operator can therefore bend the insertion section 23 in both the UD direction and the RL direction with, for example, the left hand only, while holding the sheath with the right hand and thereby positioning the insertion section 23 in a duct.

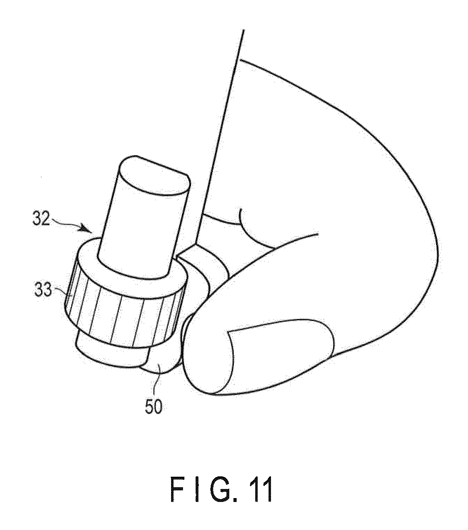

FIG. 11 shows the dial not being manipulated by the operator. The operator may place, for example, the middle finger of the left hand on the projection 50 provided in the vicinity of the RL operation member 32, while holding the grip 25 in the palm of the left hand and not turning the dial 33. This prevents the operator from touching the dial 33 unintentionally, and ultimately prevents him or her from contacting the dial 33, by mistake, to bend the bending part 23a of the insertion section 23.

Hence, with this endoscope 2, not only bending part 23a of the insertion section 23 can be more readily bent, but also the operator can be prevented from touching, by mistake, the operation device 22 to result in an erroneous manipulation thereof.

### [Fifth Embodiment]

The fifth embodiment of the invention will be described with reference to some other views of drawing. The components identical to those shown in FIG. 2 are designated by the same reference numbers and will not described in detail.

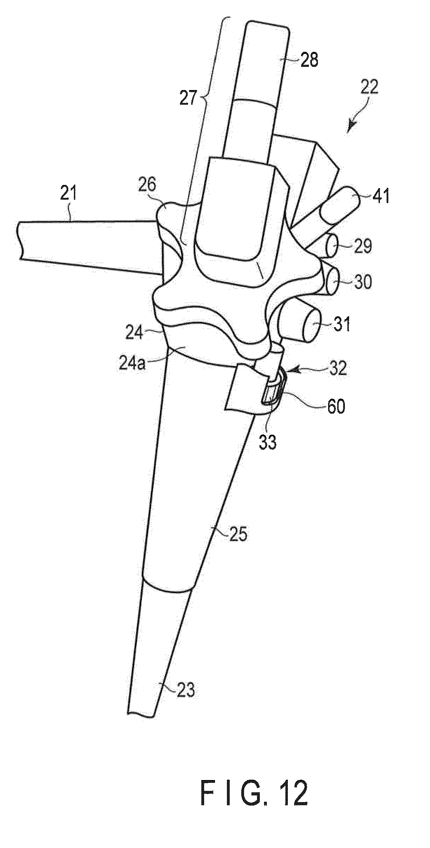

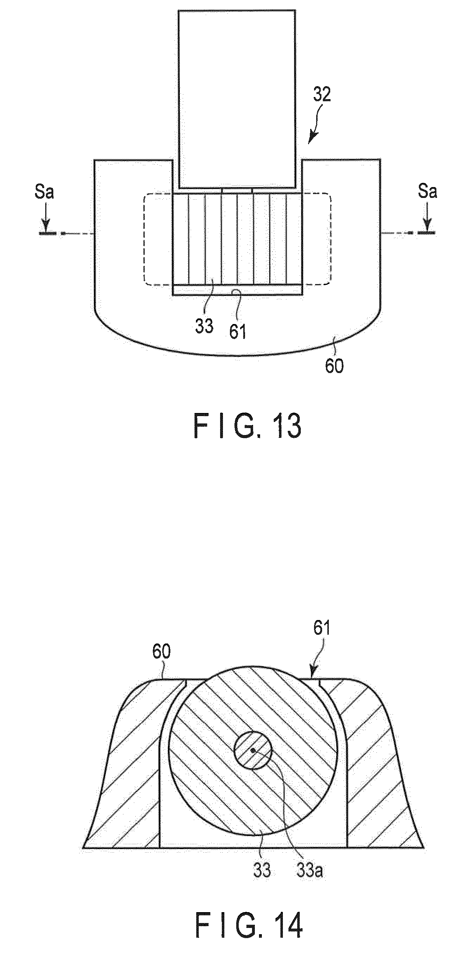

FIG. 12 shows the outer configuration of the operation device 22 of the endoscope 2. FIG. 13 is a plan view showing the error preventing unit provided on the operation member of the endoscope 2. FIG. 14 is a sectional view of the error preventing unit, taken along line Sa-Sa shown in FIG. 13.

The endoscope 2 has a projection 60 used as error preventing unit. The projection 60 is provided, covering a part of the RL operation member 32 of the operation device 22. As shown in FIG. 12, the projection 60 is provided at, for example, the boundary between the operation-unit body 24 and the grip 25. The projection 60 is arranged, covering the both sides of the dial 33 of the RL operation member 32.

More specifically, the projection 60 is arranged around the outer circumference of the dial 33 as shown in FIG. 13 and FIG. 14, covering the sides of the dial 33, which oppose each other across the shaft 33a of the dial 33. The projection 60 is so positioned not to prevent the dial 33 of the RL operation member 32 from being rotated. The projection 60 has a rectangular notch 61. The notch 61 exposes a part of the outer circumference of the dial 33, making the same protrude outwards.

So positioned, the projection 60 prevents the operator from unintentionally touching the outer circumference of the dial 33 with, for example, the middle finger, ring finger or small finger of the left hand, while holding the grip 25 with the left hand.

Since the operation device 22 has the projection 60 covering a part of the dial 33 of the RL operation member 32, the operator is prevented from unnecessarily touching the dial 33 with, for example, the middle finger of the left hand, while holding mainly the grip 25 in the palm of the left hand. Thus, the operator cannot manipulate the dial unless he or she intentionally touches the middle part of the outer circumference of the dial 33. Therefore, the projection 60 limits the operator's unintentional access to the dial 33 of the RL operation member 32, with, for example, his or her middle finger.

So configured as described above, the endoscope 2 prevents the operator from erroneously touching the dial 33 with, for example, the middle finger, to bend the bending part 23a of the insertion section 23 unintentionally. That is, as in the endoscope according to the fourth embodiment, the endoscope 2 can prevent the operator from unintentionally touching the operation device 22 to bend the bending part 23a unnecessarily.

### [Sixth Embodiment]

The sixth embodiment of the invention will be described with reference to some other views of drawing. The components identical to those shown in FIG. 2 are designated by the same reference numbers and will not described in detail.

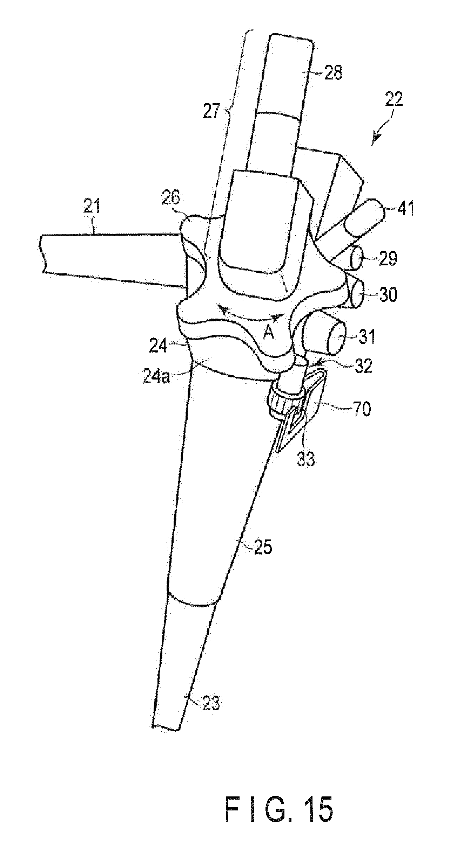

FIG. 15 shows the outer configuration of an endoscope according to the sixth embodiment of the invention. In the vicinity of the RL operation member 32, a spring cover 70 is arranged as an error preventing member. The spring cover 70 is provided, for example, at the boundary between the operation-unit body 24 and the grip 25. The spring cover 70 overlaps the outer circumference of the dial 33 of the RL operation member 32 and covers the dial 33.

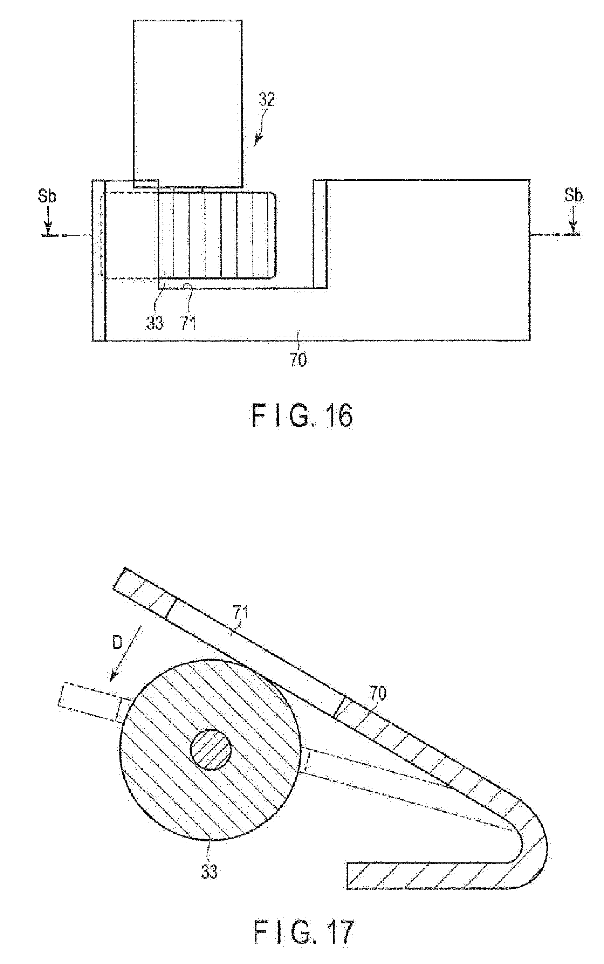

FIG. 16 is a plan view of the error preventing unit provided on the operation member. FIG. 17 is a sectional view, taken along line Sb-Sb show in FIG. 16. The spring cover 70 extends right above the dial 33. So positioned, the spring cover 70 does not impair the rotation of the dial 33 of the RL operation member 32. The spring cover 70 is a leaf spring. The spring cover 70 applies a bias away from the dial 33.

The spring cover 70 has been formed by bending a plate and has a V-shaped cross section. The spring cover 70 has a rectangular notch 71. The notch 71 is made, through which the dial 33 extends. The spring cover 70 is secured with, for examples, screws to that part of the operation-unit body 24, which lies below the air/water supply button 31.

The spring cover 70 prevents the operator from touching the dial 33 with, for example, the middle finger, ring finger or small finger of the left hand, while holding the grip 25 with the left hand.

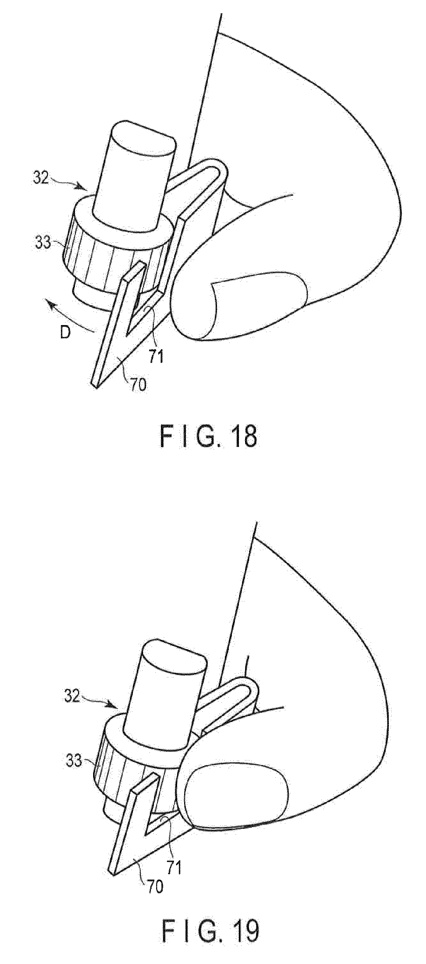

FIG. 18 shows the dial not being manipulated by the operator. The operator may push the spring cover 70 in the direction of arrow D, against the spring bias with, for example, the middle finger of the left hand, while holding the grip 25 in the palm of the left hand.

FIG. 19 shows the dial 33 being manipulated by the operator. The dial 33 protrudes from the spring cover 70 through the notch 71 made in the spring cover 70. While pushing the spring cover 70, the operator can therefore turn the dial 33 protruding outwards through the notch 71.

Since the spring cover 70 is provided, wrapping the dial 33, the operator is prevented from touching the dial 33 unnecessarily with, for example, the middle finger of the left hand, while holding the grip 25 in the palm of the left hand. Thus, unless the operator intentionally pushes the spring cover 70, the spring cover 70 prevents he or her from accessing the dial 33 with, for example, the middle finger.

Since the endoscope 2 is so configured as described above, the operator is prevented from erroneously touching the dial 33 with, for example, the middle finger, to bend the bending part 23a of the insertion section 23 unintentionally. That is, as in the endoscope according to the fourth and fifth embodiments, the endoscope 2 can prevent the operator from unintentionally touching the operation device 22 to bend the bending part 23a unnecessarily.

### [Seventh Embodiment]

The seventh embodiment of the invention will be described with reference to some other views of drawing. The components identical to those shown in FIG. 2 are designated by the same reference numbers and will not described in detail.

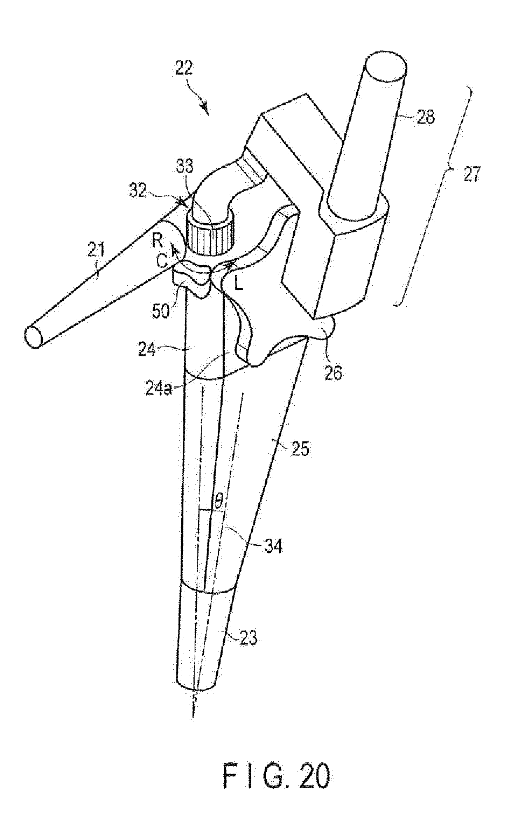

FIG. 20 shows the outer configuration of an endoscope according to the seventh embodiment of the invention. The RL operation member 32 is provided on the operation-unit body 24, above the UD angle knob 26 and on the left side of the UD angle knob 26. Also in the operation device 22 of this embodiment, a projection 50 of the type used in the fourth embodiment is arranged. The projection 50 may be replaced by the projection 60 used in the fifth embodiment or by the spring cover 70 used in the sixth embodiment.

The RL operation member 32 is provided in a manipulation area, where the operator can manipulate it with, for example, mainly the thumb of the left hand, while holding the grip 25 in the palm of the left hand. On the operation-unit body 24, the RL operation member 32 may be provided above the UD angle knob 26 and near the right of the UD angle knob 26. Alternatively, the RL operation member 32 may be provided in a manipulation area, where the operator can manipulate it with, for example, mainly the forefinger of the left hand, while holding the grip 25 in the palm of the left hand.

If manipulated by the operator, the RL operation member 32 drives the RL drive motor 28, thereby automatically bending the insertion section 23 in the UD direction. The RL operation member 32 has a dial 33. The dial 33 is shaped like, for example, a disc, as shown in FIG. 9. The dial 33 is provided, able to rotate in the direction of arrow C, around a shaft 33c passing through the centers of the circular surfaces of the dial 33, which oppose each other.

A potentiometer 33b is coupled to the shaft 33a of the dial 33. The potentiometer 33b outputs an electric signal of a magnitude equivalent to the angle by which the dial 33 has been rotated in the direction of arrow C. The RL drive unit 27 receives the electric signal from the potentiometer 33b and drives the RL drive motor 28 in accordance with the electrical magnitude of the electric signal. The insertion section 23 is thereby bent in the RL direction. If the dial 33 rotated clockwise as viewed from above, from the operation-unit body 24 toward the grip 25, the bending part 23a will be bent the R direction. If the dial 33 is rotated counterclockwise, the bending part 23a will be bent in the L direction.

The dial 33 has its shaft 33c inclined by angle θ to the axis 34 of the operation-unit body 24 and grip 25. Inclined by angle θ to the axis 34, the shaft 33c gradually leaves the axis 34 of the operation-unit body 24 and grip 25, as it extends from the grip 25 toward the operation-unit body 24. The inclination angle θ is set, ranging from 7° to 10°, for example. The optimal value for the inclination angle θ is set to, for example, 8.5°, particularly in the case where the operator rotates the dial 33, while holding the grip 25 with one hand. The dial 33 protrudes, in part, from the surface of the operation-unit body 24.

The operator takes the endoscope 2 so configured, holding the grip 25 in the palm of his or her left hand. The operator then rotates the UD angle knob 26 with, for example, the thumb in the direction of arrow A. As the UD angle knob 26 is so rotated, the bending part 23a of the insertion section 23 is bent in the UD direction. The operator further rotates the dial 33 of the RL operation member 32 with, for example, the thumb in the direction of arrow C, while holding the grip 25 with one hand. Still further, the operator turns the UD angle knob 26 in the direction of arrow A, with the thumb only, and may also rotate the dial 33 with the thumb in the direction of arrow C. The operator then operates the switch 29, the suction button 30 or the air/water supply button 31 with, for example, the forefinger.

If the dial 33 is turned, the potentiometer 33b coupled to the haft 33a of the dial 33 outputs an electric signal of the magnitude equivalent to the angle by which the dial 33 has been rotated in the direction of arrow B. The RL drive unit 27 receives the electric signal from the potentiometer 33b and drives the RL drive motor 28 in accordance with the electrical magnitude of the electric signal. The bending part 23a of the insertion section 23 is thereby bent in the RL direction.

Thus, even if the operator is not so much experienced in operating the endoscope 2, he or she can manipulate the UD angle knob 26 and also rotate the dial 33 of the RL operation member 32 in the same way as a skilled operator, while holding the operation device 22 with one hand, as with the endoscope 2 according to the fourth embodiment. The operator can therefore bend the bending part 23a of the insertion section 23 not only in the UD direction, but also in the RL direction, at the same time.

With this endoscope 2, the insertion section 23 can be readily bent in both the UD direction and the RL direction. This enables an unskilled operator to bend the endoscope 2 in the UD direction and RL direction, in the same way as skilled operators do. The operator can therefore bend the insertion section 23 in both the UD direction and the RL direction with, for example, the left hand only, while holding the sheath with the right hand and thereby positioning the insertion section 23 in a duct.

### [Eighth Embodiment]

The eighth embodiment of the invention will be described with reference to some other views of drawing. The components identical to those shown in FIG. 2 are designated by the same reference numbers and will not described in detail.

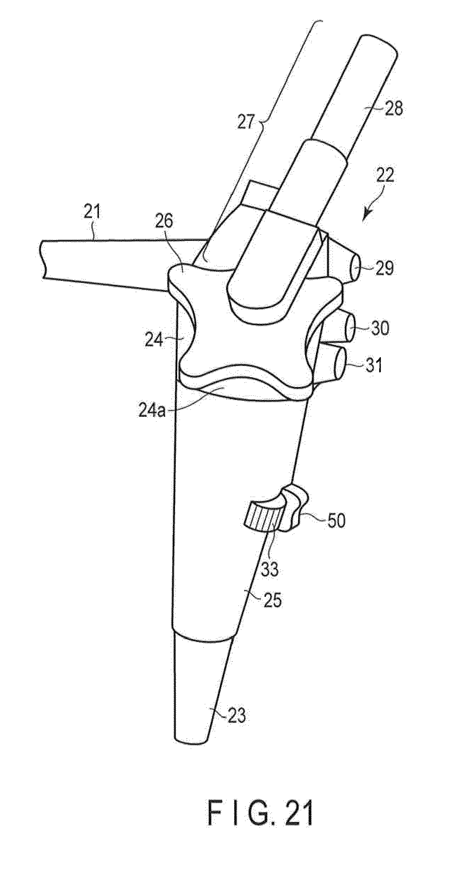

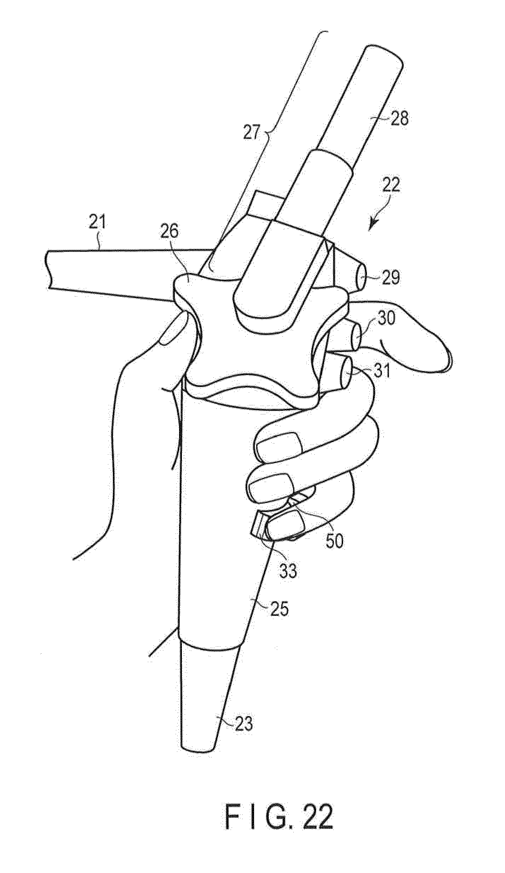

FIG. 21 shows the outer configuration of an endoscope according to the eighth embodiment of the invention. FIG. 22 shows the outer appearance of the endoscope of FIG. 21, which an operator is holding with one hand.

The RL operation member 32 is provided on the grip 25, in such an operating area that the operator may manipulate the dial 33 with, for example, the small finger of the left hand, while holding the grip 25 with the left hand and manipulating the UD angle knob 26 with, for example, the thumb of the left hand. The RL operation member 32 is provided on the grip 25, at the midpoint between the upper and lower ends of the grip 25, and below the switch 29, suction button 30 and air/water supply button 31 which are aligned in the vertical direction. Alternatively, the RL operation member 32 may be provided on that part of the grip 25, which lies between the row of the switch 29, suction button 30 and air/water supply button 31 and a frontal part of the operation-unit body 24.

Also in the operation device 22 of this embodiment, a projection 50 of the type used in the fourth embodiment is arranged. The projection 50 may be replaced by the projection 60 used in the fifth embodiment or by the spring cover 70 used in the sixth embodiment.

If manipulated by the operator, the RL operation member 32 drives the RL drive motor 28, thereby automatically bending the bending part 23a of the insertion section 23 in the UD direction. The RL operation member 32 has a dial 33. A potentiometer 33b is coupled to the shaft 33a of the dial 33. The potentiometer 33b outputs an electric signal of a magnitude equivalent to the angle by which the dial 33 has been rotated in the direction of arrow B. The dial 33 protrudes, in part, from the surface of the operation-unit body 24.

The operator takes the endoscope 2 so configured, holding the grip 25 in the palm of his or her left hand. The operator then rotates the UD angle knob 26 with, for example, the thumb in the direction of arrow A. As the UD angle knob 26 is so rotated, the bending part 23a of the insertion section 23 is bent in the UD direction. The operator further rotates the dial 33 of the RL operation member 32 with, for example, the small finger in the direction of arrow D, while holding the grip 25 with one hand. Still further, the operator turns the UD angle knob 26 in the direction of arrow A, with the thumb only, and also rotate the dial 33 of the RL operation member 32 with the small finger in the direction of arrow D. The operator then operates the switch 29, the suction button 30 or the air/water supply button 31 with, for example, the forefinger.

If the dial 33 is turned, the potentiometer 33b coupled to the haft 33a of the dial 33 outputs an electric signal of the magnitude equivalent to the angle by which the dial 33 has been rotated in the direction of arrow D. The RL drive unit 27 receives the electric signal from the potentiometer 33b and drives the RL drive motor 28 in accordance with the electrical magnitude of the electric signal. The bending part 23a of the insertion section 23 is thereby bent in the RL direction.

Thus, even if the operator is not so much experienced in operating the endoscope 2, he or she can manipulate the UD angle knob 26 and also rotate the dial 33 of the RL operation member 32 in the same way as a skilled operator, while holding the operation device 22 with one hand, as with the endoscope 2 according to the fourth embodiment. The operator can therefore bend the bending part 23a of the insertion section 23 not only in the UD direction, but also in the RL direction, at the same time.

With this endoscope 2, the insertion section 23 can be readily bent in both the UD direction and the RL direction. This enables an unskilled operator to bend the endoscope 2 in the UD direction and RL direction, in the same way as skilled operators do. The operator, even if not so skilled, can therefore bend the insertion section 23 in both the UD direction and the RL direction with, for example, the left hand only, while holding the sheath with the right hand and thereby positioning the insertion section 23 in a duct.

This invention may be modified as follows.

The dial 33 used as RL operation member 32, for example, may be provided on the operation-unit body 24. On operation-unit body 24, the dial 33 may be provided in an manipulation area where the operator can turn the dial 33 with, for example, the forefinger, middle finger, ring finger or small finger of the left hand, while holding the grip 25 with the left hand and manipulating the UD angle knob 26 with the thumb of the left hand.

Additional advantages and modifications will readily occur to those skilled in the art. Therefore, the invention if its broader aspects is not limited to the specific details and representative embodiments shown and described herein. Accordingly, various modifications may be made without departing from the spirit or scone of the general inventive concept as defined by the appended claims and their equivalents.

## Claims

1. An endoscope **characterized by** comprising:
an insertion section including a bending part capable of bending in a first direction and a second direction orthogonal to the first direction;
a first bending mechanism configured to bend the bending part in the first direction;
a drive unit configured to generate a drive force for bending the bending part in the second direction;
a second bending mechanism configured to transmit the drive force to the bending part, thereby to bend the bending part in the second direction;
an operation-unit body configured to bend the bending part;
a grip formed integral with the operation-unit body and configured to be held with a hand;
an angle mechanism provided on the operation-unit body and configured to set, to the first direction, a first operation input for bending the bending part in the first direction, and configured to transmit the first operation input to the first bending part if the first operation input is input;
an operation member provided on the grip or the operation-unit body and configured to direct, in the second direction, an input direction of a second operation input for bending the bending part in the second direction; and
a control unit configured to output a control signal for bending the bending part in the second direction, to the to the drive unit, in response to the second operation input.

2. The endoscope according to claim 1,
**characterized in that** the operation member is provided at the grip located below the angle mechanism, assuming that the operation-unit body is provided above the grip and that the grip is provided below the operation-unit body.

3. The endoscope according to claim 2,
**characterized in that** the operation member is provided in an manipulation area to be manipulated, mainly with the forefinger, middle finger, ring finger or small finger of the hand.

4. The endoscope according to claim 1,
**characterized in that** the operation member is provided on a surface opposed to another surface across an axis extending through the operation-unit body and the grip, the other surface being touched with the palm of the hand while the hand is holding the grip.

5. The endoscope according to claim 1,
**characterized in that** the operation member is provided above the angle mechanism, on the left or right of the angle mechanism, assuming that the operation-unit body is provided above the grip and that the grip is provided below the operation-unit body.

6. The endoscope according to claim 5,
**characterized in that** the operation member is provided in the manipulation area to be manipulated, mainly with the thumb of the hand.

7. The endoscope according to claim 1,
**characterized in that** the angle mechanism is able to set a vertical direction as the first direction, and
the operation member is able to set a left/right direction as the second direction.

8. The endoscope according to claim 7,
**characterized in that** the setting the vertical direction, performed by the angle mechanism, is a main operation, and the setting the left/right direction, performed by the operation member, is an auxiliary operation.

9. The endoscope according to claim 7,
**characterized in that** the operation member includes a dial shaped like a disc and able to rotate, and a motion in the left/right direction is adjusted by an angle by which the dial is rotated.

10. The endoscope according to claim 9,
**characterized in that** the dial has a shaft around which to rotate, and the shaft inclines to an axis extending through the operation-unit body and the grip.

11. The endoscope according to claim 10,
**characterized in that** the shaft of the dial inclines, gradually leaving the axis extending through the operation-unit body and the grip, from the grip toward the operation-unit body, assuming that the operation-unit body is provided above the grip and that the grip is provided below the operation-unit body.

12. The endoscope according to claim 10,
**characterized in that** the shaft of the dial inclines by 7° to 10°.

13. The endoscope according to claim 10,
**characterized in that** the shaft of the dial inclines by 8.5°.

14. The endoscope according to claim 10,
**characterized in that** the dial is able to rotate around the axis extending through the operation-unit body or the grip.

15. The endoscope according to claim 9,
**characterized in that** the dial protrudes, in part, from a surface of the operation-unit body or a surface of the grip.

16. The endoscope according to claim 1, **characterized by** further comprising;
an error preventing unit provided near the operation member, configured to prevent the finger from touching the operation member against an operator's will.

17. The endoscope according to claim 16,
**characterized in that** the angle mechanism includes a first angle mechanism and a second angle mechanism,
the first angle mechanism provided in an manipulation area of the operation-unit body, where the operator manipulate the operation member with a finger of one hand, while holding the grip with the hand, and configured to bend the bending part in the first direction, and
the second angle mechanism provided in the manipulation area and configured to bend the bending part in the second direction.

18. The endoscope according to claim 16,
**characterized in that** the operation member includes a dial shaped like a disc and able to rotate, and the error preventing unit is a projection juxtaposed to the dial.

19. The endoscope according to claim 16,
**characterized in that** the operation member includes a dial shaped like a disc and able to rotate,
the error preventing unit is a projection covering the dial, and
the projection has a notch exposing a part of the dial.

20. The endoscope according to claim 16,
**characterized in that** the operation member includes a dial shaped like a disc and able to rotate,
the error preventing unit includes a leaf spring extending right above the dial, and
the leaf spring has a notch through which the dial protrudes when the leaf spring is pushed toward the dial.
